Europäisches Patentamt

(19) European Patent Office

Office européen des brevets

(11) Numéro de publication: **0 072 298**
**B1**

(12) **FASCICULE DE BREVET EUROPEEN**

(45) Date de publication du fascicule du brevet: **30.04.86**

(21) Numéro de dépôt: **82401415.3**

(22) Date de dépôt: **29.07.82**

(51) Int. Cl.⁴: **A 61 K 7/13**

(54) **Préparation cosmétique extemporanée à pouvoir colorant, en particulier à usage capillaire.**

(30) Priorité: **03.08.81 FR 8115024**

(43) Date de publication de la demande:
**16.02.83 Bulletin 83/07**

(45) Mention de la délivrance du brevet:
**30.04.86 Bulletin 86/18**

(84) Etats contractants désignés:
**AT BE CH DE FR GB IT LI LU NL SE**

(56) Documents cités:
**EP-A-0 018 717**
**FR-A-2 473 310**
**GB-A-2 050 166**
**US-A-4 061 602**
**US-A-4 183 366**
**US-A-4 190 064**

(73) Titulaire: **PIERRE FABRE S.A.**
**125, rue de la Faisanderie**
**F-75116 Paris (FR)**

(72) Inventeur: **Jeanjean, Michel**
**71, rue d'Aillot**
**F-81000 Castres (FR)**
Inventeur: **Azam, Anne-Marie**
**Galibran**
**F-81100 Castres (FR)**
Inventeur: **Navarro, Roger**
**Chemin des Farguettes Lambert**
**F-81100 Castres (FR)**

(74) Mandataire: **Martin, Jean-Jacques et al**
**Cabinet REGIMBEAU 26, Avenue Kléber**
**F-75116 Paris (FR)**

Courier Press, Leamington Spa, England.

# 0 072 298

**Description**

La présente invention concerne de nouvelles préparations cosmétiques, à base d'extraits naturels colorants, destinées à teinter les kératines humaines et principalement celles du cheveu. Les préparations cosmétiques selon l'invention sont des préparations extemporanées, c'est-à-dire du type se présentant sous la forme de deux compositions séparées destinées à être mélangées juste avant leur utilisation.

Il existe actuellement sur le marché de multiples produits cosmétiques ayant pour fonction de teindre les cheveux. Leur pouvoir colorant est important et indiscutable dans la variété des nuances obtenues. Mais la plupart de ces compositions procèdent de réactions chimiques drastiques entre des substances colorantes obtenues par voie de synthèse chimique et la kératine du cheveu. De telles réactions doivent être le plus souvent interrompues ou maîtrisées par un agent "neutralisant" ou un agent "inhibiteur de réaction". Compte tenu de la nature même des principes actifs colorants et des autres substances chimiques habituellement employées, il existe pour l'utilisateur un risque non négligeable de réactions cutanées orthoergiques ou allergiques (problèmes des touches d'essai à réaliser avant emploi de ces teintures).

Les préparations, objet de la présente invention, dans lesquelles le principe actif colorant utilisé est un extrait standardisé d'origine végétale, permettent précisément d'éviter ces inconvénients principalement liés à l'utilisation, dans la technique antérieure, de principes colorants à base de molécules chimiques de synthèse.

Les préparations cosmétiques extemporanées à pouvoir colorant, en particulier à usage capillaire, conforme à la présente invention, du type se présentant sous la forme de deux compositions séparées qui sont destinées à être mélangées juste avant leur utilisation sont caractérisées en ce que la première composition est constituée par une solution stable standardisée d'extrait colorant d'origine naturelle, et en ce que la seconde composition est constituée par un support comprenant un agent dispersant choisi parmi l'eau déminéralisée, un alcool comportant jusqu'à 5 atomes de carbone, un glycol comportant jusqu'à 5 atomes de carbone et les mélanges de ces composés, un agent gélifiant à base d'un dérivé cationisé de gomme de Guar présent à raison d'une concentration d'environ 0,1 à 10% en poids et de préférence d'environ 0,5 à 3% en poids de la préparation finale reconstituée, ainsi qu'un agent acidifiant organique.

Divers tests et essais de toxicité et de tolérance cutanée ou muqueuse effectués sur des animaux de laboratoire, ainsi que des tests de tolérance à l'usage de l'homme ont clairement démontré la parfaite inocuité des extraits colorants et surtout des préparations objet de la présente invention.

D'autres caractéristiques et avantages des préparations cosmétiques selon l'invention apparaîtront à la lecture de la description détaillée faite ci-après, notamment en référence à quelques exemples indiqués à simple titre d'illustration de l'objet de la présente invention.

Un autre avantage des teintures obtenues avec les préparations objet de la présente invention, réside dans le fait que le cheveu n'a nul besoin d'être dénaturé ou décoloré initialement, ou encore soumis à un quelconque traitement préalable. En revanche, seul un simple lavage avant l'application de la préparation est conseillé afin d'éliminer les salissures des cheveux.

La teinture est obtenue à l'aide des préparations cosmétiques selon l'invention par le dépôt, autour de la tige capillaire, d'une sorte de gaine colorée externe, avec accrochage renforcé par la nature de certains constituants de l'excipient. La fonction détaillée de cet excipient sera précisée ci-après plus en détail. Le rôle des constituants du support, son pH, ses additifs sont primordiaux pour potentialiser le colorant lui-même et conduire aux nouvelles préparations objet de la présente invention. Il a de plus été constaté de façon surprenante que les nouvelles préparations de l'invention confèrent de plus aux chevelures ainsi traitées un pouvoir démêlant et coiffant particulier, ainsi qu'une brillance et un éclat remarquables.

Les préparations cosmétiques extemporanées selon la présente invention se présentent sous la forme de deux compositions, chacune contenue dans un récipient mono-dose, associées dans un emballage commun. Le premier récipient A contient une solution stabilisée de l'extrait colorant naturel, le deuxième récipient B un support cosmétologiquement acceptable renfermant des catalyseurs ou substances potentialisatrices et autres additifs dont le rôle essentiel sera développé plus en détail ci-après. Après mélange du contenu des flacons A et B, l'application se fait raie par raie, puis de la racine sur la totalité de la chevelure. Après un certain temps de pause la chevelure est rincée à l'eau et les cheveux séchés (avec ou sans mise en plis). Bien évidemment l'utilisateur pourra avantageusement se munir de gants afin d'éviter toute coloration des mains.

La première composition de la préparation cosmétique extemporanée selon l'invention, est constituée par la solution colorante proprement dite. Il s'agit en fait d'une solution stable standardisée d'extraits colorants d'origine naturelle. Ces extraits végétaux naturels sont obtenus extractivement, sous forme de solutions stables et standardisées.

De nombreuses plantes ont ainsi été étudiées dans le cadre de l'élaboration de la présente invention. En particulier, les extraits issus des plantes indiquées ci-après ont été jugés préférés pour leurs performances esthétiques et leurs qualités cosmétiques qu'ils ont conféré aux préparations selon l'invention:

2

— Camomille (matricaria chamomilla)
— Curcuma (curcuma longa)
— Henné (lawsonia inermis)
— Santal (pterocarpus indicus)
— Rocou (bixa orellana)
— Orcanette (alcana tinctoria)

Il est bien clair que la liste des principes actifs colorants indiqués ci-dessus ne doit nullement être considérée comme limitative, d'autres extraits végétaux naturels pouvant évidement entrer dans la composition des préparations cosmétiques selon l'invention. Il est également bien sûr évident que ces divers extraits peuvent être utilisés soit seuls, soit associés entre eux à des concentrations variables dans le but d'obtenir des nuances et des coloris variés.

Dans cette première composition, le ou les principes actifs colorants végétaux sont dissous dans un milieu dispersant qui peut être avantageusement constitué par des alcools ou des glycols de bas poids moléculaire seul ou en mélange entre eux, ou encore en mélange avec de l'eau. On peut ainsi envisager des milieux dispersants glycoliques, alcooliques, alcoolo-glycoliques, hydroglycoliques, hydro-alcooliques ou hydro-alcoolo-glycoliques, les alcools et les glycols présents dans ce milieu dispersant ayant de préférence un bas poids moléculaire, c'est-à-dire contenant de préférence jusqu'à 5 atomes de carbone.

Les solutions stables standardisées d'extraits colorants d'origine naturelle ainsi réalisés permettent d'assurer une meilleure stabilité du colorant, ainsi qu'une parfaite conservation des qualités et propriétés intrinsèques de l'extrait jusqu'au moment de son utilisation. Le conditionnement en flacons "mono-dose", avantageusement effectué sous atmosphère d'azote, garantit de plus une excellente stabilité au produit en évitant tous risques de contamination microbienne et fongique ou encore tout autre risque lié à des phénomènes d'oxydation.

On notera enfin que la concentration globale en principes colorants végétaux peut varier dans de très larges mesures, des résultats parfaitement satisfaisants ayant été obtenus dans la pratique avec une concentration en principes colorants végétaux sensiblement comprise entre 2 et 20% en poids de la préparation cosmétique totale reconstituée.

La seconde composition, également présentée en flacon mono-dose constitue en fait le support de la préparation selon l'invention et bénéficie également d'excellentes garanties de stabilité et d'intégrité de conservation dans le temps.

Il importe de remarquer ici que ce support n'est pas un simple véhicule ou excipient d'une composition cosmétologique. En effet, il participe effectivement à l'opération de teinture du cheveu de par la nature spécifique de ses constituants. Il a en effet été constaté qu'il existait entre l'action de la solution stable standardisée d'extraits colorants naturels (précédemment décrite) appliquée seule et l'action de la préparation obtenue par mélange extemporané des constituants A et B, une différence absolue de nature dans le résultat obtenu. Pareille différence a été observée aussi bien du strict point de vue de la coloration, de sont intensité de la durée de sa tenue etc., que des qualités conférées au cheveu ainsi traité (brillance, gainage, tenue, failité de coiffage, etc.).

La seconde composition constituant le support est globalement composée par un milieu dispersant dans lequel ont été introduits d'une part un agent gélifiant et filmogène spécifique d'origine naturelle modifiée, et d'autre part un agent acidifiant organique de préférence également d'origine naturelle.

De façon classique, pour le traitement de certains types de cheveux (par exemple très secs ou encore très abîmés), le support peut également contenir un agent conditionneur substantif supplémentaire.

Il convient de noter ici qu'un très grand nombre de véhicules classiquement utilisées en cosmétologie capillaire ont été essayés en vain dans le cadre de l'application à la préparation cosmétique selon l'invention. Certains d'entre eux inhibent purement et simplement les propriétés tinctoriales des extraits, d'autres présentent des incompatibilités physicochimiques telles que le colorant n'est absolument pas fixé et se trouve donc éliminé soit au rinçage, soit très rapidement après l'usage. Parmi les produits testés négativement on citera les agents suivants:

. tensio-actifs ou émulsifiants non ioniques (alkanolamides, esters gras éthoxylés, . . .,)
. tensio-actifs anioniques (alkyls sulfates, alkyl éther sulfates, alkyl sulfo-succinates, alkyl polypeptides), et
. tensio-actifs amphotères (alkyl bétaïnes, dérivés imidazolines).

On notera de surcroît que les émulsions se sont également avérées être un mauvais véhicule, car si l'extrait peut être facilement émulsionné avec des corps gras, il perd immédiatement toutes ses propriétés tinctoriales.

La seconde composition constituant le support comprendra en premier lieu un agent dispersant, véhicule cosmétologiquement acceptable, qui peut être constitué par l'eau déminéralisée, un alcool à bas poids moléculaire, un glycol à bas poids moléculaire ou encore un mélange de ces composés. L'expression "à bas poids moléculaire" désigne des composés comportant jusqu'à 5 atomes de carbone. On notera que dans la pratique, les solutions hydroalcooliques ont donné des résultats tout particulièrement satisfaisants. Le degré alcoolique de telles solutions peut être avantageusement compris entre 5 et 40°, et de préférence entre 10 et 20°.

3

**0 072 298**

Il a en effet été observé que l'alcool, associé à l'eau et/ou à un glycol, facilite la dispersion du colorant sur la tige capillaire, tout en respectant l'intégrité kératinique de cette dernière. Il a également été observé qu'un degré alcoolique trop élevé s'avère fortement inhibiteur de la fixation de la couleur sur le cheveu.

Il provoque même un dessèchement important du cheveu et bloque d'une façon importante les effets substantifs de l'agent gélifiant entrant également dans la composition du support. De même le milieu dispersant pourra totalement ou partiellement être constitué par un extrait végétal de Saponines en association éventuelle avec un ou plusieurs véhicules dispersants évoqués.

L'agent gélifiant a été rigoureusement et scientifiquement sélectionné pour son affinité kératinique particulière et ses effets cosmétiques sur le cheveu. Cet agent gélifiant et filmogène est constitué par un dérivé d'ammonium quaternaire de farine de gomme Guar. Ce dérivé d'ammonium peut être constitué avantageusement par un sel de trialkylammonium et de préférence un halogénure de trialkylammonium. L'essentiel des expérimentations a été effectué avec un halogénure, et de préférence le chlorure de triméthylammonium hydroxypropylique de farine de gomme Guar.

La gomme Guar est obtenue par broyage de l'albumen des graines de Cyamopsis tetragonolobus (papilionacée cultivée en Inde). Elle est principalement constituée par un galactomannane, dit guarane. Cette gomme naturelle a subi une réaction chimique assurant sa transformation sous forme cationisée ou quaternisée.

Le chlorure de triméthylammonium hydroxypropylique de farine de Guar est par exemple commercialisé par la Société Henkel sous la dénomination "Cosmédia Guar C 261".

Le dérivé cationisé de farine de gomme Guar est avantageusement présent dans la préparation selon l'invention à raison d'une concentration de 0,1 à 10% en poids, et de préférence de 0,5 à 3% en poids, de la préparation finale reconstituée. On notera enfin que la fonction spécifique de l'agent gélifiant intervenant dans la composition du support est en fait double, puisque ce dernier agit comme:

— agent gainant: il permet en effet une répartition homogène du colorant à la surface des cheveux et aide à la fixation du colorant.
— agent conditionneur: il assure un coiffage aisé du cheveu mouillé, ainsi qu'une souplesse et une tenue parfaite du cheveu sec. De plus, la brillance qu'il confère aux cheveux après séchage accentue encore la coloration obtenue après application de la préparation selon l'invention.

Lors de l'élaboration de la présente invention, de nombreux agents gélifiants ont été testés vainement, puisque seuls des dérivés cationisés de farine de gomme Guar se sont révélés posséder les deux fonctions de gainage et de conditionnement précédemment évoqués. En effet, avec tous les autres agents gélifiants classiques testés (résines polyacryliques, dérivés de silice tels que silices colloïdales et silicates, dérivés de cellulose tels qu'esters éthylique et méthylique, diverses gommes Arabique, Xanthane, Carraghénanes, etc.), il apparaissait une très nette discontinuité, étant donné que tous ces agents gélifiants ne conduisaient qu'à des préparations très imparfaites.

Le support contient enfin un agent acidifiant organique. Cet agent permet bien sûr l'ajustement du pH du support puis de la solution dans la zone où les conditions de résultats se trouvent être les meilleurs (pH compris entre 3 et 6 et plus particulièrement entre 3 et 4,5 pour la préparation finale à l'état reconstitué).

Cependant l'agent acidifiant organique remplit de plus deux autres fonctions essentielles, à savoir:

— il agit comme mordant fixateur du principe colorant sur la tige kératinique, et
— il potentialise le pouvoir conditionneur, démêlant et substantif du mélange sur le cheveu. Cet agent organique acidifiant est avantageusement choisi parmi l'acide alginique, l'acide galacturonique, l'acide citrique ainsi que leur mélange. Cet agent organique acidifiant est avantageusement présent à raison d'une concentration de 0,1 à 4% en poids et de préférence de 0,5 à 2% en poids du support.

Dans le choix particulier de ce dernier constituant du support, à savoir l'agent acidifiant organique, de nombreuses recherches ont également été effectuées sur une gamme étendue d'acides organiques. L'acide tannique, l'acide gallique, l'acide tartrique et l'acide malique ont également permis d'obtenir des résultats tout à fait acceptables ainsi que leurs sels. Toutefois, les meilleurs résultats ont été obtenus avec les acides alginique, citrique, galacturonique et un extrait aqueux total de citron, ainsi qu'avec les couples acide alginique+acide citrique, acide alginique+extrait aqueux total de citron et acide galacturonique+acide citrique. Les sels des acides précités ont permis également d'obtenir d'excellents résultats.

A titre d'illustration de la mise en oeuvre des préparations cosmétologiques selon l'invention, on mentionnera ci-après un exemple particulier de mode d'utilisation qui a conduit dans la pratique à d'excellents résultats.

On chauffe légèrement au bain marie jusque vers 40°C les deux récipients "mono-dose" A et B contenant respectivement la solution de colorant et le support gélifié et acidifié. Lorsque la température est satisfaisante, on procède au mélange extemporané.

Sur les cheveux préalablement lavés et essorés (ou sur chevelure propre et mouillée afin de faire gonfler la kératine), on applique la préparation avec un système approprié (par exemple un tube canule) d'abord près des racines, puis en faisant glisser le produit vers l'extrémité des cheveux.

4

On vérifie que toute la chevelure a bien été imprégnée, puis on laisse agir, la tête étant coiffée d'un bonnet spécial, à température ambiante, pendant la durée recommandée pour la teinte particulière à obtenir (ces conditions sont fonction et de la composition choisie et de la teinte de départ de la chevelure).

On procède ensuite à un simple rinçage à l'eau tiède. Ce dernier peut être suivi d'un simple séchage, ou bien d'une mise en plis ou d'un "brushing".

On mentionnera également ci-après quelques exemples non limitatifs de préparations selon l'invention.

### Exemple 1

| | | |
|---|---|---|
| A)—extrait de Lawsonia inermis | | 15% |
| solution hydro-glycolique (20—80) q.s.p. | | 100 ml |
| conditionnement en flacon verre de | | 15 ml |

| | | |
|---|---|---|
| B)—gomme cationisée | | 1,5% |
| acide citrique | | 0,5% |
| solution hydro-alcoolique (85—15) q.s.p. | | 100 ml |
| conditionnement en flacon plastique de | | |
| 100 ml rempli par 50 ml de solution B. | | |

### Exemple 2

| | | |
|---|---|---|
| A)—extrait de Pterocarpus indicus | | 15% |
| extrait de Lawsonia inermis | | 5% |
| solution glyco-alcoolique (80—20) q.s.p. | | 100 ml |
| conditionnement en ampoule verre | | |
| monopointe de 15 ml | | |

| | | |
|---|---|---|
| B)—gomme gélifiante | | 1% |
| silicate de magnésium et d'aluminium | | 2% |
| acide alginique | | 0,7% |
| acide citrique | | 0,3% |
| solution aqueuse q.s.p. | | 100 ml |
| conditionnement en flacon de 100 ml | | |
| rempli par 50 ml de solution B. | | |

### Exemple 3

| | | |
|---|---|---|
| A)—extrait de Matricaria Chamomilla | | 10% |
| extrait de Curcuma Longa | | 0,5% |
| solution glycolique q.s.p. | | 100 ml |
| conditionnement en flacon verre de 20 ml | | |

| | | |
|---|---|---|
| B)—gomme gélifiante cationisée | | 2% |
| extrait aqueux de Citrus limonum | | 1,5% |
| solution hydro-alcoolique (88—12) q.s.p. | | 100 ml |
| conditionnement comme pour les exemples | | |
| 1 et 2 ci-dessus. | | |

### Exemple 4

| | | |
|---|---|---|
| A)—extrait d'Alcana tinctoria | | 8% |
| extrait de Pterocarpus indicus | | 12% |
| solution hydro-glycolique (30—70) q.s.p. | | 100 ml |
| conditionnement comme dans l'exemple 3 | | |

| | | |
|---|---|---|
| B)—gomme gélifiante cationisée | | 1,25% |
| polymère cationique conditionneur | | 0,30% |
| extrait aqueux de Citrus Limonum | | 1% |
| acide de galacturonique | | 0,20% |
| solution hydro-alcoolique (90—10) q.s.p. | | 100 ml |
| conditionnement comme dans les exemples | | |
| ci-dessus. | | |

Il est parfaitement clair que les préparations cosmétiques selon la présente invention ne sauraient être limitées aux exemples particuliers décrits. En revanche, il est parfaitement possible, sans pour autant sortir du cadre de la présente invention, d'en imaginer un certain nombre de variantes et de modifications. C'est ainsi que les préparations selon l'invention peuvent contenir d'autres constituants tels que par exemple un agent de démêlage. En effet, conjointement au dérivé cationisé de gomme Guar, il peut être introduit dans

5

la formulation, un agent démêlant par substantivité, de structure cationisée ou non dont l'affinité pour la tige capillaire renforce encore l'action du premier.

Tout comme lui, il permet une répartition uniforme de la couleur sur le cheveu, de la racine vers la pointe tout en assumant son rôle principal qui est de démêler et de conditionner. La concentration d'un tel agent démêlant pourra varier entre 0,1 et 5% en poids de la formulation selon la nature chimique du produit utilisé, c'est-à-dire en fonction de ses capacités de fixation sur le cheveu.

Parmi les produits de ce type testés associativement avec la gomme Guar cationisée, on peut par exemple citer les classes de composés suivants:

— Ammoniums quaternaires
— Pseudo-cationiques polymérisés ou non
— Résines type vinylique
— Dérivés protéiniques quaternisés
— Dérivés polysiloxanes éthoxylés.

## Revendications

1. Préparation cosmétique extemporanée à pouvoir colorant, en particulier à usage capillaire, du type se présentant sous la forme de deux compositions séparées qui sont destinées à être mélangées juste avant leur utilisation, caractérisée en ce que la première composition est constituée par une solution stable standardisée d'extrait colorant d'origine naturelle, et en ce que la seconde composition est constituée par un support comprenant un agent dispersant choisi parmi l'eau déminéralisée, un alcool comportant jusqu'à 5 atomes de carbone, un glycol comportant jusqu'à 5 atomes de carbone et les mélanges de ces composés, un agent gélifiant à base d'un dérivé cationisé de gomme de Guar présent à raison d'une concentration d'environ 0,1 à 10% en poids et de préférence d'environ 0,5 à 3% en poids de la préparation finale reconstituée, ainsi qu'un agent acidifiant organique.

2. Préparation selon la revendication 1, caractérisée en ce que la première composition comprend un ou plusieurs principes colorants végétaux dissous dans un milieu dispersant glycolique, alcoolique, alcoolo-glycolique, hydro-glycolique, hydro-alcoolique ou hydro-alcoolo-glycolique.

3. Préparation selon la revendication 2, caractérisée en ce que le ou les principes colorants végétaux sont présents à une concentration d'environ 2 à 20% en poids de la préparation cosmétique totale reconstituée.

4. Préparation selon la revendication 1, caractérisée en ce que ledit agent dispersant présente un degré alcoolique sensiblement compris entre 5 et 40° et de préférence entre environ 10 et 20°.

5. Préparation selon l'une des revendications 1 à 4, caractérisée en ce que ledit agent gélifiant à base d'un dérivé cationisé de farine de gomme Guar est constitué par un dérivé d'ammonium quaternaire de farine de gomme Guar.

6. Préparation selon la revendication 5, caractérisée en ce que le dérivé d'ammonium de farine de gomme Guar est constitué par le sel de trialkylammonium, de préférence un halogénure de trialkylammonium.

7. Préparation selon la revendication 6, caractérisée en ce que l'halogénure de trialkylammonium de farine de gomme Guar est constitué par un halogénure, de préférence le chlorure de triméthylammonium hydroxy-propylique de farine de gomme Guar.

8. Préparation selon l'une des revendications 1 à 7, caractérisée en ce que ledit agent organique acidifiant est choisi parmi l'acide alginique, l'acide galacturonique, l'acide citrique ainsi que leurs mélanges.

9. Préparation selon la revendication 8, caractérisée en ce que ledit agent organique acidifiant est présent à raison d'une concentration d'environ 0,1 à 4% en poids et de préférence d'environ 0,5 à 2% en poids du support.

## Patentansprüche

1. Kosmetische, nach dem Mischen sofort zu verwendende Zubereitung mit Färbewirkung, insbesondere zur Anwendung für Haare, in der Form von zwei getrennten Zusammensetzungen, die zum Zusammenmischen unmittelbar vor ihrer Anwendung bestimmt sind, dadurch gekennzeichnet, daß die erste Zusammensetzung aus einer beständigen, standardisierten Lösung eines färbenden Auszuges natürlichen Ursprungs besteht, und daß die zweite Zusammensetzung aus einem ein aus demineralisiertem Wasser, einem Alkohol mit bis zu 5 Kohlenstoffatomen, einem Glykol mit bis zu 5 Kohlenstoffatomen und Gemischen dieser Verbindungen ausgewähltes Dispergens enthaltenden Trägermaterial, einem Gelierungsmittel auf der Basis eines kationischen Derivates von Guargummi, in einer Konzentration von etwa 0,1 bis 10 Gew.-% und vorzugsweise 0,5 bis 3 Gew.-% der endgültigen fertigen Zubereitung sowie einem organischen Säuerungsmittel besteht.

2. Zubereitung nach Anspruch 1, dadurch gekennzeichnet, daß die erste Zusammensetzung ein oder mehrere in einem dispergierenden, glykolischen, alkoholischen, alkoholisch-glykolischen, wäßrig-

6

glykolischen, wäßrig-alkoholischen oder wäßrig-alkoholisch-glykolischen Medium aufgelöste pflanzliche färbende Prinzipien enthält.

3. Zubereitung nach Anspruch 2, dadurch gekennzeichnet, daß das oder die pflanzlichen färbenden Prinzipien in einer Konzentration von etwa 2 bis 20 Gew.-% der gesamten fertigen kosmetischen Zubereitung vorhanden sind.

4. Zubereitung nach Anspruch 1, dadurch gekennzeichnet, daß das Dispergens einen alkoholischen Grad von im wesentlichen 5 bis 40° und vorzugsweise von 10 bis 20° aufweist.

5. Zubereitung nach einem der Ansprüche 1 bis 4, dadurch gekennzeichnet, daß das Geliermittel auf der Basis eines kationischen Derivates von Guargummimehl aus einem quaternären Ammoniumderivat von Guargummimehl besteht.

6. Zubereitung nach Anspruch 5, dadurch gekennzeichnet, daß das Ammoniumderivat von Guargummimehl aus einem Trialkylammoniumhalogenid, vorzugsweise einem Trialkylammoniumhalogenid, besteht.

7. Zubereitung nach Anspruch 6, dadurch gekennzeichnet, daß das Trialkylammoniumhalogenid von Guargummimehl aus einem Halogenid, vorzugsweise dem Hydroxypropyltrimethylammoniumchlorid von Guargummimehl besteht.

8. Zubereitung nach einem der Ansprüche 1 bis 7, dadurch gekennzeichnet, daß das organische Säuerungsmittel aus Alginsäure, Galacturonsäure, Zitronensäure sowie deren Gemischen ausgewählt ist.

9. Zubereitung nach Anspruch 8, dadurch gekennzeichnet, daß das organische Säuerungsmittel in einem Konzentrationsverhältnis von etwa 0,1 bis 4 Gew.-% und vorzugsweise von etwa 0,5 bis 2 Gew.-% des Trägermaterials vorhanden ist.

**Claims**

1. An extemporaneous cosmetic preparation with dyeing capability, in particular for capillary use, of the type presented in the form of two separate compositions which are designed to be mixed together just before use, characterised in that the first composition consists of a standardized stable solution of a dyeing extract of natural origin, and in that the second composition consists of a carrier comprising a dispersing agent selected from deionised water, an alcohol having up to 5 carbon atoms, a glycol having up to 5 carbon atoms and mixtures of these compounds, a gelling agent based on a cationized derivative of guar gum present in a concentration of from about 0.1 to 10% by weight and preferably from about 0.5 to 3% by weight of the final reconstituted preparation, as well as an organic acidifying agent.

2. A preparation according to claim 1, characterised in that the first composition comprises one or more vegetable dyeing principles dissolved in a glycolic, alcoholic, alcoholic-glycolic, aqueous-glycolic, aqueous-alcoholic or aqueous-alcoholic-glycolic dispersing medium.

3. A preparation according to claim 2, characterised in that the vegetable dyeing principle(s) is/are present in a concentration of from about 2 to 20% by weight of the total reconstituted cosmetic preparation.

4. A preparation according to claim 1, characterised in that the said dispersing agent has a percentage of alcohol of substantially between 5 and 40°, preferably between about 10 and 20°.

5. A preparation according to one of claims 1 to 4, characterised in that the said gelling agent based on a cationized derivative of guar gum flour consists of a quaternary ammonium derivative of guar gum flour.

6. A preparation according to claim 5, characterised in that the ammonium derivative of guar gum flour consists of a trialkylammonium salt, preferably a trialkylammonium halide.

7. A preparation according to claim 6, characterised in that the trialkylammonium halide of guar gum flour consists of a halide, preferably the trimethylammonium hydroxypropyl chloride of guar gum flour.

8. A preparation according to one of claims 1 to 7, characterised in that the said organic acidifying agent is selected from alginic acid, galacturonic acid, citric acid and mixtures thereof.

9. A preparation according to claim 8, characterised in that the said organic acidifying agent is present in a concentration of from about 0.1 to 4% by weight, preferably from about 0.5 to 2% by weight of the carrier.